# EUROPEAN PATENT SPECIFICATION

(11) **EP 2 464 969 B1**
(45) Date of publication and mention of the grant of the patent: **25.12.2013**
(21) Application number: 10745197.3
(22) Date of filing: 10.08.2010
(51) Int. Cl.: G01N 33/50

(54) **NANOPARTICLE SCREENING METHODS EMPLOYING INSECTS WITH BLOOD BRAIN BARRIER**
NANOPARTIKEL-SCREENINGVERFAHREN MIT INSEKTEN MIT BLUT-HIRN-SCHRANKE
PROCÉDÉS DE CRIBLAGE DE NANOPARTICULES EMPLOYANT DES INSECTES À BARRIÈRE HÉMATO-ENCÉPHALIQUE

(30) Priority: 12.08.2009 US 233154 P
(43) Date of publication of application: 20.06.2012
(73) Proprietor: Entomopharm APS, 5250 Odense SV (DK)
(72) Inventor: AADAL NIELSEN, Peter, S-23840 Oxie (SE); ANDERSSON, Gunnar, S-26024 Roestaanga (SE); ANDERSSON, Olga, S-26024 Roestaanga (SE)
(74) Representative: Orsnes, Henrik Egede
(86) International application number: PCT/EP2010/061596
(87) International publication number: WO 2011/018451

(56) References cited:
- MOKRI-MOAYYED BEHZAD ET AL: "Development of a novel ex vivo insect model for studying virulence determinants of Escherichia coli K1." JOURNAL OF MEDICAL MICROBIOLOGY JAN 2008 LNKD- PUBMED:18065675, vol. 57, no. Pt 1, January 2008 (2008-01), pages 106-110, XP002601667 ISSN: 0022-2615
- FISCHER ET AL: "Nanotoxicity: the growing need for in vivo study" CURRENT OPINION IN BIOTECHNOLOGY, LONDON, GB LNKD- DOI:10.1016/J.COPBIO.2007.11.008, vol. 18, no. 6, 21 December 2007 (2007-12-21), pages 565-571, XP022409624 ISSN: 0958-1669
- MAYER FAHIMA ET AL: "Evolutionary conservation of vertebrate blood-brain barrier chemoprotective mechanisms in Drosophila." THE JOURNAL OF NEUROSCIENCE : THE OFFICIAL JOURNAL OF THE SOCIETY FOR NEUROSCIENCE 18 MAR 2009 LNKD- PUBMED:19295159, vol. 29, no. 11, 18 March 2009 (2009-03-18), pages 3538-3550, XP002601668 ISSN: 1529-2401
- HAMAMOTO H ET AL: "Silkworm as a model animal to evaluate drug candidate toxicity and metabolism" COMPARATIVE BIOCHEMISTRY AND PHYSIOLOGY PART C: TOXICOLOGY & PHARMACOLOGY, ELSEVIER LNKD- DOI:10.1016/J.CBPC.2008.08.008, vol. 149, no. 3, 1 April 2009 (2009-04-01) , pages 334-339, XP025988606 ISSN: 1532-0456 [retrieved on 2008-09-06]

## Description

### FIELD OF THE INVENTION

The present invention is directed to insect models that are aimed to reflect vertebrate blood-brain barrier (BBB) penetration of nanoparticles. Investigation of BBB penetration of nanoparticles is extremely important since there is an increasing use of nanoparticles and the profile for many of these are yet to be understood. Moreover, nanoparticles are important in drug discovery as they have proven to be useful as carriers for potential CNS drugs. On the one hand successful CNS drugs have to cross the BBB. On the other hand BBB penetration of nanoparticles may also cause unwanted side effects. Specifically, the present invention relates to the use of insects in screening for BBB penetration of nanoparticles.

### BACKGROUND OF THE INVENTION

Although there are anecdotal data indicating a causal relationship between long-term ultrafine particle exposures in ambient air (e.g., traffic related) or at the workplace (e.g., metal fumes) and resultant neurotoxic effects in humans, more studies are needed to test the hypothesis that inhaled nanoparticles (NP) or NPs absorbed via food cause neurodegenerative effects. Some NPs may have a significant toxicity (hazard) potential, and this will pose a significant risk if there is a sufficient exposure. The challenge is to identify such hazardous NPs and take appropriate measures to prevent exposure.

Also in relation to fighting CNS brain disorders NPs have shown promising as carriers of drugs that could not otherwise have passed the blood brain barrier. The brain is quite unique from other organs of the body. Despite enormous advances in brain research, CNS brain disorders still remain accountable for a high number of hospitalizations requiring prolonged care. It is estimated that approximately 1.5 billion people worldwide are suffering from various CNS disorders, such as Alzheimer's disease, Parkinson's disease, amyotrophic lateral sclerosis, multiple sclerosis, HIV-dementia and stroke, among others. In recent years, the explosion of interest in the biological aspects of CNS disorders as well as the brain has led to an improvement in the understanding and the treatment of these diseases; however, they still remain chronic and debilitating disorders for many patients. As demographic trends show an increase in the percentage of elderly people in the population, diseases of the brain are becoming a major health problem. It is expected that the number of cases of CNS diseases will be around 1.9 billion by 2020. Despite these challenges, brain drug targeting plays a vital role and gives tremendous hope for the treatment of these disorders.

The blood-brain barrier (BBB) has always presented a challenge to scientists for brain drug targeting. The BBB has evolved in such a way that it protects the brain from various foreign substances such as neurotoxins. This mechanism makes the BBB an insurmountable barrier for numerous highly essential drugs, including antibiotics, cytostatics and other CNS-active drugs.

Many strategies have been developed to overcome the hurdles caused by the BBB. They include both invasive and noninvasive approaches. The invasive approaches include the temporary disruption of the BBB, which allows the entry of drugs to the brain, and direct drug delivery to the brain by means of intraventricular or intracerebral injections, and intracerebral polymeric implants. The noninvasive approaches use colloidal drug carriers.

Among the noninvasive approaches, polymeric nanoparticles, especially poly(butylcyanoacrylate) (PBCA) nanoparticles coated with polysorbate 80, have recently received much attention from neuroscientists as an attractive and innovative carrier for brain targeting. These nanoparticles may be defined as 'a submicron drug-carrier system, which are generally polymeric in nature. Since nanoparticles are small in size, they easily penetrate into small capillaries and can be taken up within cells, allowing efficient drug accumulation at targeted sites in the body. The first reported nanoparticles were based on nonbiodegradable polymeric systems. Their use for systemic administration, however, could not be considered because of the possibility of chronic toxicity due to the tissue and immunological response towards the nondegradable polymer. Hence, nanoparticles prepared with biodegradable polymers such as poly(cyanoacrylate) were exclusively studied. The use of biodegradable materials for nanoparticle preparation allows sustained drug release at the targeted site over a period of days or even weeks after injection.

Certain insects may be suitable as model organisms for studying BBB penetration of NPs. Insects are multi cell organisms with complex compartmentalized nervous systems for specialized functions like vision, olfaction, learning, and memory. The nervous systems of the insects respond physiologically in similar ways as in vertebrates with many identical neurohormones and receptors. Insects have avascular nervous systems in which hemolymph bathes all outer surfaces of ganglia and nerves. Therefore, many insects require a sophisticated BBB system to protect their CNS from plant-derived neurotoxins and to maintain an appropriate ionic microenvironment of the neurons. In fact, also in insects a sophisticated BBB system has been an evolutionary advantage. In insects this BBB is mainly based on the glia cell system which certainly shifted to the endothelial system as a response to the increased importance of the microvasculature in the vertebrate brain. In support of this view is the appearance of the glia system in elasmobranch fish and the remnants of their glia barrier in modern mammalian CNS. Thus insects possess a BBB which is an important component in the ensheathment of the nervous system. The BBBs in insects are highly sophisticated but varies in structure between different insect orders. Thus insects with highly sophisticated brain barriers with complex integrative components that mimic the vertebrate barriers will be excellent models for documentation of penetration of various molecules through this structure.

US20050132425A1 discloses a transgenic fly that expresses the Italian mutant version of the human Abeta42 peptide of human amyloid-beta precursor protein (APP), and a double transgenic fly that expresses both the Tau protein and the human Abeta42_{Italian} peptide of human amyloid-beta precursor protein (APP). The transgenic flies provide for models of neurodegenerative disorders, such as Alzheimer's disease. US20050132425A1 further discloses methods for identifying genetic modifiers, as well as screening methods to identify therapeutic compounds to treat neurodegenerative disorders using the transgenic flies.

WO04006854A2 discloses a method for screening for the effect of a test agent on a population of insects comprising the steps of providing a population of specimen, administering at least one test agent to the population, creating a digitized movie showing the movements of the insects, measuring at least one trait of the insects of the population with the effect of the test agent. The document also provides a method for preparing a medicament useful for the treatment of a mammalian disease.

Marsh and Thompson (Neuron - 2006 - 52:169-178) teach that insects are very useful as model systems due to simplicity combined with fast reproducibility. Some of the models (with Drosophila) have demonstrated their efficiency for testing relevant drugs and revealed concordance of drug efficacy in flies and mammals for diseases like Huntingtons's, Parkinson's and Alzheimer's.

Marsh and Thompson (BioEssays - 2004 - 26:485-496) suggest that the dominant neurodegenerative diseases of man can be faithfully modelled in insects, such as Drosophila (fruitfly), since they exhibit the key features of these diseases such as slowly progressing degeneration, late onset, formation of abnormal protein aggregates etc. According to the authors the ability to manipulate such engineered organisms allows pathogenic mechanisms to be identified and potential pharmacologic regimens to be rapidly tested. The authors suggest that the excellent agreement to date of pharmacologic treatments that are effective in suppressing pathology in both flies and in mice gives growing confidence that invertebrate model organisms can productively speed the identification of agents that are likely to be effective at treating diseases in mammals.

Mokri-Moayyed et al (J. Med. Microbiol., 2008, 57: 106-110) discloses a method for determining the transport of *E.coli* K1 across the blood brain barrier (BBB) comprising injecting bacteria to a locust, incubating the insects and dissecting brains from the insects. The authors do not mention nano-particles.

There is an urgent need for more sophisticated screening models in drug discovery but also in testing of CNS toxicity of chemicals on market with less known effects on brain function.

Thus, in drug discovery there is a need for efficient screening of NPs aimed at targets within the CNS system. This screening is preferentially performed in insect models with intact BBB function and will contribute to a positive selection of compounds penetrating the vertebrate BBB.

There is also an increasing need for an efficient environmental safety screening model that can be used to identify the potential hazardous nanoparticles (from the environment), which enter the brain.

Hence, there is a demand for intermediate models that are more reliable than *in vitro* models and at the same time faster and cheaper than traditional vertebrate *in vivo* models.

### SUMMARY OF THE INVENTION

In one aspect the present invention is directed to the use of insects for studying the environmental safety of nanoparticles. The particles are administered into the hemolymf of an insect having a BBB, such as the locust, and the uptake into the brain is analysed. Thus, in this model it is possible to investigate the potential up-take into the brain of nanoparticles circulating in the blood.

The overall object of the present invention is to develop insect screening models to determine blood-brain barrier penetration of different NPs. This object offers many advantages relative to prior technologies since insect models are more reliable tools for the decision-making process than the existing *in vitro* models, and will speed up the screening process and reduce the late phase attrition rate. Moreover, it will reduce the number of mammals sacrificed during the drug discovery phase.

The present inventors have surprisingly found that the blood-brain barrier (BBB) in insects selected from the group consisting of cockroaches, locusts and moths has more in common with BBB of mammals than previously assumed. The insects of the present invention may therefore serve as an intermediate model for determination of BBB penetration of NPs.

The present invention is also able to provide for the first time rational strategies for screening NPs carrying compounds for neurological indications, as well as generating a simple in vivo system for determining NPs brain penetration.

Drug discovery is a long and costly process, requiring vast amount of chemical and biological resources. In the present invention the possibilities to use insects as model systems have been thoroughly exploited in order to improve the decision processes and reduce the costs during the drug discovery phase. Based on recent discoveries the inventors have fully contemplated that insect models of the present invention provide a better foundation than the existing *in vitro* models for selection of compounds to be tested in vertebrates.

In one aspect, the invention provides a method for screening for the BBB penetration of a nanoparticle, said method comprises the steps of:
administering the nanoparticle to insects selected from the orders consisting of Blattodea, Acridoidea, Cheleutoptera, Brachycera and Lepidoptera,
incubating the insects for a period of between 1 minute and 1 month
dissecting brains from the insects,
measuring the concentration of the test agent in the dissected brains.

In a preferred embodiment of the present invention the insects are selected from the Acridoidea (locusts) and Blattodea (cockroaches) orders.

The dissection of the brains should preferably be performed immediately after sacrificing the insects. Alternatively, the brains are dissected and removed from living insects.

Preferably the dissected brains are homogenized or disintegrated by ultra sound and eventually lysed in order to obtain a homogeneous liquid reflecting the composition of the brains. The liquid is centrifuged and the supernatant stored until analysis. The further analysis of the liquid may be performed by virtue of liquid chromatography, possibly with mass spectrometric detection of the eluted compounds. Alternatively, the presence of NPs in the brain may be indentified and quantified by histochemical methods.

In various aspects and embodiments the present invention provides the subject-matter set out in the claims below.

### DETAILED DESCRIPTION OF THE INVENTION

The present invention provides new methodology for screening for BBB penetration of nanoparticles, which enter the blood stream. The invention is generally useful for medium to high throughput screening for nanoparticles developed in drug discovery programs targeting a variety of diseases and disorders, specifically degenerative disorders, including: Parkinson's Disease, Alzheimer's Disease, Huntington's Disease, Diseases with motor neuron inclusions, Tauopathies, Corticobasal degeneration Neuropsychiatric disorders, including: Depression Bipolar disease, Schizophrenia, Anxiety, Aggression, and Brain tumours.

A nanoparticle in accordance with the present invention is a particle sized between 1 and 100 nanometers. There are several methods for creating nanoparticles, including both attrition and pyrolysis. In attrition, macro or micro scale particles are ground in a ball mill or other size reducing mechanism. A thermal plasma can also deliver the energy necessary to cause evaporation of small micrometer size particles. Inert-gas condensation is frequently used to make nanoparticles from metals with low melting points. The metal is vaporized in a vacuum chamber and then supercooled with an inert gas stream. The supercooled metal vapor condenses into nanometer-sized particles, which can be entrained in the inert gas stream and deposited on a substrate or studied in situ.

The present invention relates to but is not restricted to the use of insects selected from the following orders: (Taxonomy according to: Djurens Värld, Ed B.Hanström; Förlagshuset Norden AB, Maölmö, 1964):

| **Order** | **Suborder/family** | **Comment** |
|---|---|---|
| Dictyoptera | Blattoidea | Cockroach |
| | Mantoidea | |
| Orthoptera | Grylloidea | Crickets |
| | Acridoidea | Grasshoppers |
| Cheleutoptera | | Stick insects |
| Lepidoptera | | Moths |
| Hymenoptera | Formicoidea | Ants |
| | Vespoidea | Wasps |
| | Apoidea | Bee like Hymenopterans |
| | Bombinae | Bumble-bees |
| | Apine | Proper bees |
| Odonata | | Dragonflies |
| Diptera | Nematocera | Mosquitos |
| | Brachycera | Flies E.g Drosophila |

In particular the invention relates to insect species selected from Blattoidea, Acridoidea, Cheleutoptera, Brachycera and Lepidoptera and most particular to the Acridoidea (Locusta migratoria and Schistocera gregaria).

The invention will also relate to the following orders comprising insect species relevant for the method of the present invention:

| **Order** | **Suborder/family** | **Comment** |
|---|---|---|
| Ephemerida | | Mayflies |
| Plecoptera | | |
| Dermoptera | Forficuloidea | Earwigs |
| Homoptera | Cicadinea | Cicadas |
| | Aphidine | Plant-louse |
| Heteroptera | | Hemipteran |
| Coleoptera | | Beetles |
| Trichoptera | | Caddis fly |

The present invention preferably uses large insects, such as the migratoty locust, Locusta migratoria and the desert locust, Schistocera gregaria or cockroach where it is feasible to feed and inject nanoparticles and subsequently take hemolymph samples and dissect brain tissues, for analyses. The locust has been used to develop screening models to compare this model with existing literature data from conventional *in vivo* vertebrate studies.

In accordance with a preferred embodiment of the present invention the migratoty locust, Locusta migratoria and/or the desert locust, Schistocera gregaria, is used since it is easy to breed and it is a relatively large insect (40 - 60 mm long, weight: approx. 2 g, hemolymph volume: approx. 300 µL, brain weight: approx. 2 mg).

The application of nanoparticles to insects of the present invention in a screening method may be as follows, in accordance with a preferred embodiment of the present invention.

### EXAMPLES

In a preferred embodiment of present invention the insects are selected from the order Acridoidea and specifically Locusta migratoria and Schistocera gregaria are used. The insects may be obtained from local suppliers or bred in house. The grasshoppers were reared under crowded conditions at 28° C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. Nanoparticles are administrated into the haemolymph in a way similar to that described by Goldworthy et al. (2003). For quantitative determination of brain nanoparticle concentration the brains are dissected (a cut was made through the frontal part of the locust head comprising the most frontal parts including the antennae, the compound eyes, the brain and all neural connections between the brain and the antennae and the eyes), washed, snap-frozen and stored until analyses. At analysis the brains are homogenised/vortexed and centrifuged. The nanoparticle containing supernatant is analysed for its drug concentration by HPLC, LC/MSMS or other relevant methods. The effect of drug treatment may be documented by recording the pharmacological effects on behaviour or by use of recordings of central nervous system nerve signalling.

In the following the present invention is exemplified in further detail.

### Example 1

Amine modified fluorescent polystyrene nanoparticles (100 nm) were injected (20 ul) into the hemolymph of locusts (Locusta migratoria). After 60 minutes hemolymph was harvested and analyzed for uptake of nanoparticles by hemolymph cells. There was a highly selective uptake of the nanoparticles into macrophage like cells and no accumulation in other cell types. It was also found that the particles were accumulated into the lysosomes strongly indicating that the particles were taken up by endocytosis.

Conclusion: The uptake of nanoparticles into endocytic cells of the locust hemolymph is identical with the in vivo uptake of nanoparticles by vertebrate macrophages (Sadauskas et al., 2007). This observation is important since the clearance of nanoparticles from the circulation by macrophages is very efficient (about 90% at 60 minutes after administration in a mouse study) but also dependent on the shape of the nanoparticle and this clearance mechanism is therefore a determinant of the exposure of the nanoparticle at the brain barrier.

### Example 2.

Silver nanoparticles (SNP; 80 nm) were injected into the hemolymph of locusts. After 3 and 24 hours the locust brains were dissected in saline, the neural lamella removed and the brains washed twice in saline and then placed in tubes containing 100 ul nitric acid. The content of Ag was analyzed by ICP-MS. At 3 hours there was an average uptake of 0.58 ng Ag per brain and at 24 hours the uptake had increased to 0.86 ng Ag per brain. Average background content of Ag in control brains was 0.36 ng per brain.

### Example 3.

A cut was made through the frontal part of the head of female locusts. Each brain in its cuticle was placed in a SNP (80 nm) solution for 3 hours. The brains were dissected in saline, the neural lamella removed, washed twice in saline and then placed in tubes containing 100 ul nitric acid. The average content of Ag per per brain was 4.82 ng as measured by ICP-MS.

### Example 4.

A cut was made through the frontal part of the head of female locusts. Each brain in its cuticle shell was placed in a SNP (80 nm) solution for 3 hours. The brains were dissected in saline, the neural lamella removed, washed twice in saline and then placed in tubes containing 100 ul nitric acid. The average content of Ag per per brain was 6.40 ng as measured by ICP-MS.

### Example 5.

Locust brains were dissected in saline and the neural lamella removed. The brains were placed in a SNP (80 nm) solution for 3 hours. The brains were washed twice in saline and then placed in tubes containing 100 ul nitric acid. The average content of Ag per per brain was 21.7 ng as measured by ICP-MS.

### Example 6.

Silver nanoparticles (SNP; 57 nm) was injected into the hemolymph of locusts. After 24 hours the locust brains were dissected in saline, the neural lamella removed and the brains exposed to Evans blue. There was a marked uptake of Evans blue in SNP treated locusts whereas there was no uptake in control brains.

Conclusions: Similar to the findings in vertebrates, SNP are taken up in vivo by the locust brain and thus pass the locust brain barrier. The observation of Evans blue uptake after 24 hours exposure in vivo is similar to that found for SNP in a rat model showing deterioration of the BBB integrity 24 hours after i.v administration (Sharma et al., 2010).
Ex vivo exposure markedly increased brain uptake and the uptake was further increased in the locust brain when exposed to the SNP after removal of the neural lamella.

### Example 7.

A cut was made through the frontal part of the head of female locusts. Each brain in its cuticule shell was placed in a solution of amino modified fluorescent polystyrene nanoparticles (100 nm) for 60 min. The brains were dissected in saline, washed twice in saline and then placed in tubes with paraformaldehyde. Microscopic analysis of nanoparticle fluorescence in tissue slices showed a marked uptake in the neural lamella. Conclusion. This study support the difference found in the SNP studies with and without the neural lamella at exposure to the SNP solution.

### Example 8.

Locust brains were dissected in saline and the neural lamella was removed. The brains were placed in solutions containing amino modified fluorescent polystyrene (100 nm) nanoparticles and exposed for 3 hours. The brains were then placed in tubes with paraformaldehyde.

Microscopic analysis of nanoparticle fluorescence in tissue slices showed a marked uptake in the locust brains of the 100 nm amino modified nanoparticles and there was a marked deterioration of the barrier.

### Example 9.

Locust brains were dissected in saline and the neural lamella was removed. The brains were placed in solutions containing amino modified fluorescent polystyrene (50 nm) nanoparticles and exposed for 3 hours. The brains were then placed in tubes with paraformaldehyde.
Microscopic analysis of nanoparticle fluorescence in tissue slices showed a marked uptake in the locust brain barrier region of the 50 nm amino modified nanoparticles but there was no deterioration of the barrier.

### Example 10.

Locust brains were dissected in saline and the neural lamella was removed. The brains were placed in solutions containing carboxymodified fluorescent polystyrene (30 nm) nanoparticles and exposed for 3 hours. The brains were then placed in tubes with paraformaldehyde.

Microscopic analysis of nanoparticle fluorescence in tissue slices showed no uptake in the locust brain of the 30 nm carboxy modified nanoparticles.

### General conclusion:

In vivo exposure of various nanoparticles to the locust hemolymph results in the same elimination by endocytic cell uptake and uptake over the brain barrier as in vertebrate models. Ex vivo modeling in the locust clearly indicate the utility of the model in studies of brain permeation with reference to nanoparticle size and chemical properties. The observations of effects on brain barrier integrity is of extraordinary importance since the effects of nanoparticles on brain barrier permeation due to functional deterioration is often not fully understood and also the polysorbate 80 coated PBCA nanoparticle (widely used for carrying drugs into the brain) is suggested to deteriorate the vertebrate BBB (Olivier, 2005).

### REFERENCES

Banerjee and Baht (2007), Neuron-Glial Interactions in Blood-Brain Barrier Formation Annual Review of Neuroscience 30: 235-258.
Di, L. and Kerns, E.H. (2003). Profiling drug-like properties in discovery research. Current Opinion in Chemical Biology 7,402-408.
Gaertner, L.S., Murray, C.L., Morris, C.E. (1998). Transepithelial transport of nicotine and vinblastine in isolated malpighian tubules of the tobacco hornworm (Manduca sexta) suggests a P-glycoprotein-like mechanism. The Journal of Experimental Biology 201, 2637-2645.
Garberg, P. et al. (2005). In vitro models for the blood-brain barrier. Toxicology in Vitro 19, 299-334.
Goldsworthy, G et al. (2003) Adipokinetic hormone enhances nodule formation and phenoloxidase activation in adult locusts injected with bacterial lipopolysaccaride. J. Insect. Physiol., 49, 793-803.
Gullan, P.J and Cranston, P.S. (2000). The insects. An outline of entomology. Blackwell Science Ltd.
Marsh, J.L., and Thompson, L.M. (2004). Can flies help humans treat neurodegenerative diseases? Bioessays 26, 485-496.
Marsh, J.L. and Thompson, L.M. (2006). Drosophila in the Study of Neurodegenerative Disease. Neuron 52, 169-178.
Olivier, J.C. (2005). Drug transport to brain with targeted nanoparticles. J. American Soc. Exp Neuro Therapy, 2, 108-119.
Pardridge, W.M. (2002). Drug and gene targeting to the brain with molecular Trojan horses. Nature Reviews Drug Discovery 1, 131-139
Ruiz-Garcia, A., Bermejo, M., Moss A., Casabo, V.G. (2007). Pharmacokinetics in drug discovery. Journal of Pharmaceutical Sciences, 1-37.
Sadauskas. E. et al. (2007). Kupffer cells are central in removal of nanoparticles from the organism. Particle and Fibre Toxicology 19, 4-10.
Schinkel, A.H. (1999). P-Glycoprotein, a gatekeeper in the blood-brain barrier. Advanced Drug Delivery Reviews 36, 179-194.
Sharma, H.S. et al. (2010). Influence of nanoparticles on blood-brain barrier permeability and brain edema formation in rats. Acta Neurochir. Suppl., 106, 359-364.
Summerfield, S. et al. (2005). Improving the In Vitro Prediction of In Vivo CNS Penetration: Integrating Permeability, Pgp Efflux and Free Fractions in Blood and Brain. Journal of Pharmacology And Experimental Therapeutics*.*
Turksen, K. and Troy, T.-C. (2004). Barriers built on claudins. Journal of Cell Science 117, 2435-2447.
Xia, C.Q., Xiao, G., Liu, N., Pimprale, S., Fox, L., Patten, C.J., Crespi, C.L., Miwa, G., Gan, L.-S. (2006). Comparison of Species Differences of P-Glycoproteins in Beagle Dog, Rhesus Monkey, and Human Using ATPase Activity Assays. Molecular Pharmaceutics 3 (1), 78-86.

## Claims

1. A method for determining whether a nanoparticle is transported across the blood brain barrier (BBB), said method comprises the steps of:
• administering the nanoparticle to insects having a BBB,
• incubating the insects,
• dissecting brains from the insects, and
• measuring the concentration of the nanoparticle in the brains.

2. The method of claim 1, wherein the insects are selected from the group consisting of the orders Blattoidea, Diptera, Acridoidea, Cheleutoptera, Brachycera, and Lepidoptera.

3. The method of claim 1, wherein the insects are incubated for a period of between 1 min and 1 month, before dissecting the brains from the insects with a view of quantitating the concentration of the administered nanoparticle in the brains.

4. The method of claim 1, wherein the dissection of the brains is performed immediately after sacrificing the insects.

5. The method of claim 1, wherein measuring the concentration of the nanoparticle is performed by homogenizing the dissected brains, preferably centrifuge the homogenate and analyzing the concentration of the nanoparticle in the homogenate by liquid chromatography, possibly with mass spectrometric detection of the eluted compounds.

6. The method according to any one of the preceding claims wherein the nanoparticle is administered parenterally or perorally.

7. The method according to any one of the preceding claims comprising determination of body: brain nanoparticle concentration gradients for quantitatively determining BBB penetration.

8. Use of insects selected from the group consisting of the orders Blattodea, Diptera, Acridoidea, Cheleutoptera, Brachycera and Lepidoptera to determine whether a nanoparticle is transported across the blood brain barrier.

## Patentansprüche

1. Verfahren zur Bestimmung, ob ein Nanopartikel durch die Blut-Hirn-Schranke transportiert wird, wobei das Verfahren die Schritte umfasst des:
- Verabreichens des Nanopartikels an Insekten, die eine Blut-Hirn-Schranke besitzen,
- Inkubierens der Insekten,
- Abpräparierens der Gehirne von den Insekten, und
- Messens der Konzentration des Nanopartikels in den Gehirnen.

2. Verfahren nach Anspruch 1, wobei die Insekten ausgewählt sind aus der Gruppe bestehend aus den Ordnungen Blattodea, Diptera, Acridoidea, Cheleutoptera, Brachycera und Lepidoptera.

3. Verfahren nach Anspruch 1, wobei die Insekten für einen Zeitraum zwischen 1 Minute und 1 Monat inkubiert werden, bevor die Gehirne mit dem Ziel der Quantifizierung der Konzentration des verabreichten Nanopartikels von den Insekten abpräpariert werden.

4. Verfahren nach Anspruch 1, wobei das Abpräparieren der Gehirne unmittelbar nach Opferung der Insekten erfolgt.

5. Verfahren nach Anspruch 1, wobei die Messung der Konzentration des Nanopartikels durch Homogenisieren der aufpräparierten Gehirne, vorzugsweise Zentrifugieren des Homogenisats und Analysieren der Konzentration des Nanopartikels in dem Homogenisat durch Flüssigchromatographie erfolgt, möglicherweise durch massenspektrometrischen Nachweis der eluierten Verbindungen.

6. Verfahren nach einem der vorherigen Ansprüche, wobei der Nanopartikel parenteral oder peroral verabreicht wird.

7. Verfahren nach einem der vorherigen Ansprüche, umfassend die Bestimmung von Konzentrationsgradienten der Nanopartikel zwischen Körper und Gehirn zur quantitativen Bestimmung der Durchlässigkeit der Blut-Hirn-Schranke.

8. Verwendung von Insekten, ausgewählt aus der Gruppe bestehend aus den Ordnungen Blattodea, Diptera, Acridoidea, Cheleutoptera, Brachycera und Lepidoptera, zur Ermittlung, ob ein Nanopartikel durch die Blut-Hirn-Schranke transportiert wird.

## Revendications

1. Un procédé pour déterminer si une nanoparticule est transporté à travers la barrière hémato-encéphalique (BHE), ledit procédé comprenant les étapes de:
- l'administration de la nanoparticule à des insectes ayant une BHE,
- l'incubation des insectes,
- la dissection des cerveaux des insectes, et
- la mesure de la concentration de la nanoparticule dans les cerveaux.

2. Procédé selon la revendication 1, dans lequel les insectes sont sélectionnées du groupe consistant des classes Blattoidea, Diptera, Acridoidea, Cheleutoptera, Brachycères, et les Lépidoptères.

3. Procédé selon la revendication 1, dans lequel les insectes sont incubées pendant une période comprise entre 1 min et 1 mois, avant la dissection des cerveaux des insectes en vue de la quantification de la concentration de la nanoparticule administré dans le cerveau.

4. Procédé selon la revendication 1, dans lequel la dissection des cerveaux est effectuée immédiatement après avoir sacrifié les insectes.

5. Procédé selon la revendication 1, dans lequel la mesure de la concentration de la nanoparticule est effectuée par la homogénéisation des cerveaux disséqués, de préférence de centrifuger l'homogénat et l'analyse de la concentration de la nanoparticule de l'homogénat par chromatographie en phase liquide, éventuellement avec la détection par spectrométrie de masse des composés élués.

6. Procédé selon l'une quelconque des revendications précédentes, dans lequel la nanoparticule est administré par voie parentérale ou par voie orale.

7. Procédé selon l'une quelconque des revendications précédentes, comprenant la détermination de corps: gradients de concentration de la nanoparticule du cerveau pour déterminer quantitativement le taux de pénétration de la BHE.

8. Utilisation des insectes sélectionnés du groupe consistant des classes Blattodea, Diptères, Acridoidea, Cheleutoptera, Brachycera et des Lépidoptères afin de déterminer si une nanoparticule est transporté à travers la barrière hématoencéphalique.
